(19) Europäisches Patentamt
European Patent Office
Office européen des brevets

(11) Numéro de publication : **0 087 337**
**B1**

(12) # FASCICULE DE BREVET EUROPÉEN

(45) Date de publication du fascicule du brevet : **16.04.86**

(51) Int. Cl.[4] : **C 07 D239/84**, A 61 K 31/505

(21) Numéro de dépôt : **83400232.1**

(22) Date de dépôt : **04.02.83**

(54) **Nouveaux dérivés de la pipérazinyl-2 phényl-4 quinazoline possédant des propriétés antidépressives, méthode de préparation desdits composés et médicaments en contenant.**

(30) Priorité : **08.02.82 FR 8201988**

(43) Date de publication de la demande :
**31.08.83 Bulletin 83/35**

(45) Mention de la délivrance du brevet :
**16.04.86 Bulletin 86/16**

(84) Etats contractants désignés :
**AT BE CH DE FR GB IT LI LU NL SE**

(56) Documents cités :
**US-A- 3 509 141**
**CHEMICAL ABSTRACTS, vol. 89, 1978, page 644, no. 43492s, Columbus, Ohio, USA**

(73) Titulaire : **SANOFI, société anonyme**
**40, Avenue George V**
**F-75008 Paris (FR)**

(72) Inventeur : **Biziere, Kathleen**
**6 Lotissement Rayons d'Oc**
**F-34170 Clapiers (FR)**
Inventeur : **Hallot, André**
**83 rue du Juge**
**F-34270 Saint-Gely du Fesc (FR)**
Inventeur : **Kan, Jean-Paul**
**25 Lotissement l'Olivette**
**F-34170 Clapiers (FR)**

(74) Mandataire : **Gillard, Marie-Louise et al**
**Cabinet Beau de Loménie 55, Rue d'Amsterdam**
**F-75008 Paris (FR)**

**EP 0 087 337 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des dérivés de la pipérazinyl-2 phényl-4 quinazoline, ainsi que leur méthode de préparation et leur application comme médicaments.

Des dérivés de la chloro-6 phényl-4 quinazoline substitués en position 2 par un groupe amino secondaire ou tertiaire, et présentant une activité antiinflammatoire sont décrits dans le brevet US 3 509 141.

Dans une demande antérieure EP-A-79810, la demanderesse a également décrit des dérivés de la phényl-4 quinazoline répondant à la formule générale :

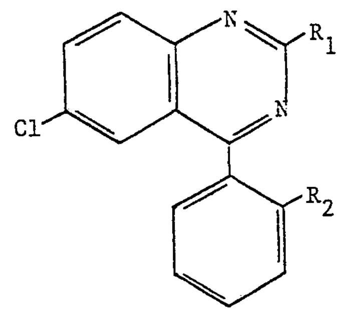

(I)

dans laquelle $R_1$ désigne un groupe amine tertiaire cyclique ou non portant lui-même un groupe hydroxyle, et $R_2$ représente un atome de chlore ou de fluor.

Les composés (I) sont doués de propriétés anticonvulsivantes et de potentialisation de la narcose permettant leur utilisation comme médicament en tant qu'anxiolytiques, hypnotiques et anti-épileptiques.

Il a été trouvé que, de façon surprenante, en faisant varier la nature du substituant $R_1$, les produits obtenus ne présentent plus les propriétés pharmacologiques des produits (I) mais sont dotés de propriétés antidépressives très intéressantes.

Les composés selon l'invention répondent à la formule générale :

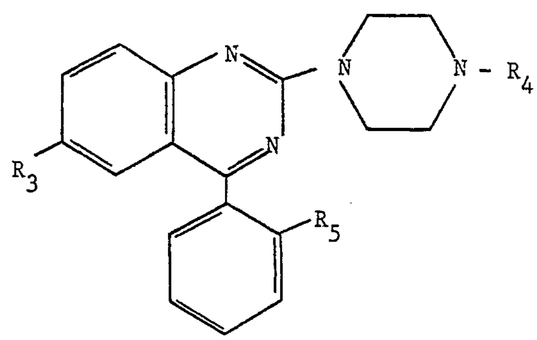

(II)

dans laquelle :

$R_3$ désigne un halogène, de préférence le chlore ou un groupe nitro,

$R_4$ représente l'hydrogène ou un groupe alkyle inférieur ayant de 1 à 4 atomes de carbone,

$R_5$ représente un atome d'halogène, de préférence le chlore ou le fluor.

Les composés (II) fournissent avec les acides minéraux ou organiques des sels solubles. Ces sels, avec des acides pharmaceutiquement acceptables, font partie intégrante de l'invention.

On notera que dans le brevet espagnol 453 982 on décrit un procédé de préparation de dérivés d'amino-2 quinazolines, substituées entre autres en position 4 par un radical phényle éventuellement substitué. Les produits obtenus selon ce procédé et décrits dans ledit brevet espagnol sont différents des produits décrits dans la présente demande ; par ailleurs, ledit brevet espagnol ne fournit aucun renseignement utilisable concernant les propriétés pharmacologiques des produits qui y sont préparés.

Les composés (II) peuvent être préparés à partir d'une phényl-4 quinazoline-2 convenablement substituée (1) suivant le schéma réactionnel :

(Voir figure p. 3)

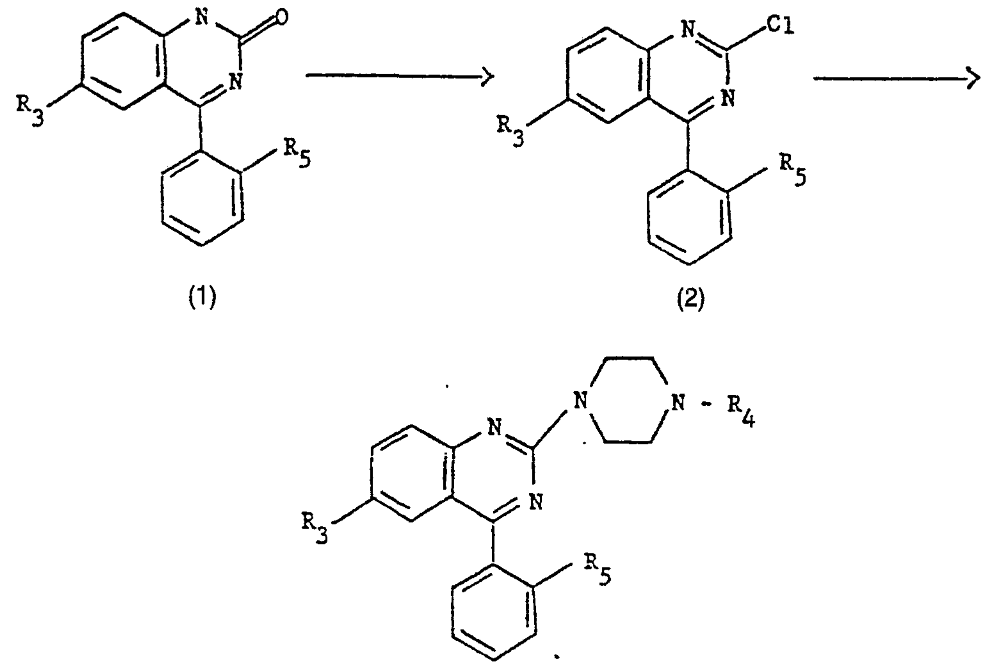

(1) (2)

(II)

Par action sur la quinazolone (1) d'un dérivé chloré du phosphore, on obtient le dérivé (2) chloré en position 2. On utilise le plus souvent l'oxychlorure de phosphore. On peut opérer au sein d'un solvant inerte tel qu'un hydrocarbure aromatique (benzène ou toluène) mais, le plus souvent, on préfère utiliser un excès d'oxychlorure comme solvant. La réaction a lieu à une température comprise entre 60 et 120 °C et le plus souvent à la température d'ébullition du solvant utilisé.

A partir du dérivé chloré (2), par action d'une amine

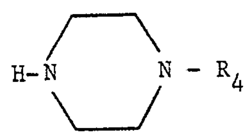

en excès au sein d'un solvant inerte comme l'éthanol, on obtient le composé (II) correspondant. On opère en général par chauffage à la température d'ébullition du solvant..

Les sels des composés (II) sont obtenus de façon habituelle en salifiant la base à chaud par une quantité stœchiométrique d'acide au sein d'un solvant convenablement choisi de façon que le sel formé cristallise par refroidissement.

Les quinazolones de départ (1) sont des composés connus. Lorsque $R_3$ = halogène, ils peuvent en particulier être préparés par action du cyanate de potassium sur une amino-2 halogéno-5 benzophénone convenablement substituée.

Lorsque $R_3$ représente le groupe nitro, les composés (1) peuvent être obtenus par action de l'urée sur une amino-2 nitro-5 benzophénone convenablement substituée.

Les exemples suivants sont donnés à titre d'illustration pour la préparation des composés (II) suivant la méthode indiquée précédemment.

Exemple 1

(Méthyl-1 pipérazinyl-4)-2 (chloro-2 phényl)-4 nitro-6 quinazoline (CM 40498)

(II) $R_3 = NO_2$ ; $R_4 = CH_3$ ; $R_5 = Cl$

a) Chloro-2 (chloro-2 phényl)-4 nitro-6 quinazoline

On chauffe à reflux pendant 4 h un mélange de 40 g de (chloro-2 phényl)-4 nitro-6 quinazolone-2 et 600 ml d'oxychlorure de phosphore. On évapore sous vide à siccité l'oxychlorure de phosphore, puis on verse le résidu dans un mélange glace-eau. On alcalinise avec une solution de soude à 10 %. On essore le précipité et on le lave avec de l'acétonitrile. On purifie le produit par chromatographie sur une colonne de silice. En éluant avec un mélange chloroforme-méthanol (95-5 volume/volume), on isole le produit attendu. Poids : 25 g ; F. : 228 °C (isopropanol).

b) CM 40498

On chauffe au reflux pendant 3 h un mélange de 3,2 g du dérivé chloré obtenu ci-dessus et 3 g de N-méthyl pipérazine dans 120 ml d'éthanol absolu. On évapore le solvant à siccité sous vide, puis on reprend le résidu dans l'acétate d'éthyle. On lave la solution avec une solution aqueuse de carbonate de sodium puis avec de l'eau. On sèche la solution sur sulfate de sodium puis évapore à siccité. On recristallise le résidu dans un mélange méthanol-dichlorométhane. On obtient des cristaux. Poids : 3,1 g ; F. : 204 °C.

Exemples 2 à 6

a) En opérant comme dans l'exemple 1a), mais en faisant varier la quinazolone, on obtient de la même façon :

— la chloro-2 (fluoro-2 phényl)-4 nitro-6 quinazoline ; F. : supérieur à 260 °C (isopropanol) ;
— la dichloro-2,6 (chloro-2 phényl)-4 quinazoline ; F. : 175-176 °C (éthanol) ;
— la dichloro-2,6 (fluoro-2 phényl)-4 quinazoline ; F. : 208-210 °C (acétonitrile).

b) A partir des divers dérivés chlorés en position 2 mentionnés précédemment et en faisant varier l'amine

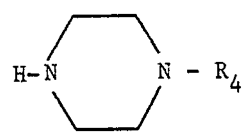

utilisée, on obtient comme indiqué dans l'exemple 1b) les différents composés (II) réunis dans le tableau I ci-après.

Les produits selon l'invention ont été soumis à des essais pharmacologiques en vue de déterminer leur activité sur le système nerveux central.

On indiquera ci-dessous les diverses épreuves auxquelles ont été soumis les produits. Dans tous les cas, les produits à étudier ont été administrés per os.

A — Test de Porsolt

Ce test a été réalisé chez la souris femelle, CDI (Charles Rivers, France) pesant 18 à 23 g, selon la méthode décrite par Porsolt (Archives Internationales de Pharmacologie, 1977, *229*, 327-336).

Le principe de ce test est le suivant : lorsqu'une souris est placée dans un récipient étroit, rempli d'eau, elle se débat puis, au bout de 2 à 4 min, s'immobilise et flotte sur le ventre, le dos arrondi, les pattes postérieures ramenées sous le corps ; elle ne fait que les quelques mouvements nécessaires pour lui maintenir la tête hors de l'eau. C'est la réaction dite de désespoir (despair reaction).

Certains psychotropes, notamment les antidépresseurs, allongent le temps pendant lequel la souris se débat.

Le protocole suivant a été choisi :

Les produits à étudier ont été administrés oralement à des lots de 10 souris. 1 h plus tard, les animaux sont placés dans un récipient étroit (10 × 10 × 10 cm), rempli d'eau sur une hauteur de 6 cm, la température de l'eau étant de 24 °C. Les animaux sont laissés 6 min dans l'eau et on mesure le temps où l'animal reste immobile entre la deuxième et la sixième minute. Plus le temps est court, plus la substance est active.

Les résultats sont exprimés comme la diminution du temps d'immobilisation par rapport aux témoins.

B — Antagonisme de la ptôse induite par la réserpine

La plupart des antidépresseurs antagonisent la ptôse induite par la rérserpine. Ce test décrit par Gouret [Journal Pharmacologie (Paris), 1973, *4* (1), 105-128], a été réalisé chez la souris femelle CDI (Charles Rivers, France) pesant 18 à 23 g. La réserpine entraîne un ptôsis 1 h après son administration intraveineuse ; certains antidépresseurs, notamment les imipraminiques, s'opposent à ce ptôsis.

Le protocole suivant a été choisi :

Les substances à étudier ont été administrées per os à des lots de 10 souris. Simultanément, la réserpine a été administrée par voie intraveineuse à la dose de 2 mg/kg. 1 h après l'administration de réserpine, on note le nombre d'animaux ne présentant pas de ptôsis.

C — Antagonisme de l'hypothermie induite par la réserpine

La plupart des antidépresseurs antagonisent l'hypothermie induite par la réserpine. Ce test a été réalisé selon la méthode décrite par HINO et Coll [Chem Pharm Bull *28* (9), 2618-2622, 1980] sur des souris femelles CDI (Charles Rivers, France) pesant 18 à 23 g.

Le protocole suivant a été choisi :

Les substances à étudier sont administrées per os à des lots de 10 souris, les témoins reçoivent le solvant seul, simultanément, la réserpine est administrée i. p. à la dose à 5 mg/kg. La température de chaque animal est prise immédiatement avant l'administration des produits à étudier et 4 h après. Pour chaque animal, la différence de température avant et après traitement est calculée. Les résultats sont exprimés en pourcentage d'antagonisme de l'hypothermie observée chez les témoins.

D — Potentialisation de la toxicité de la yohimbine

La plupart des antidépresseurs potentialisent la toxicité de la yohimbine. Ce test a été réalisé selon la méthode décrite par MALICK (in Antidepressants : Neurochimical Behavioural and Clinical Perspectives, eds S.J. ENNA, J.B. MALICK, E. RICHELSON, Raven Press, New York, pages 141-155) sur des souris femelles CDI (Charles Rivers, France).

Les substances à étudier sont administrées per os à des lots de 10 souris. La yohimbine est administrée i. p. plus tard à la dose de 30 mg/kg. La mortalité est relevée 18 h plus tard.

C — Antagonisme des tremblements à l'oxotrémorine

Les effets cholinergiques de l'imipramine sont considérés comme responsables de certains effets secondaires gênants de cette substance. Ces effets sont mis en évidence par l'antagonisme des tremblements à l'oxotrémorine. Ce test a été réalisé sur des souris femelles CDI (Charles Rivers, France) pesant 18 à 23 g.

Le protocole suivant a été choisi :

Les produits à étudier sont administrés au temps 0 par voie orale à des lots de 10 souris. Au temps 60 min, l'oxotrémorine est administrée per os à la dose de 1 mg/kg. On note le nombre de souris qui ne tremblent pas 30 min après l'administration d'oxotrémorine.

L'ensemble des résultats pharmacologiques obtenus avec divers composés selon l'invention est rassemblé dans le tableau II ci-après. Dans ce tableau, figurent également les résultats obtenus, d'une part, avec l'imipramine, composé dont les propriétés antidépressives sont largement utilisées en thérapeutique et, d'autre part, avec le composé CM 40 331 ou chlorhydrate de chloro-6 (chloro-2 phényl)-4 (hydroxy-4 pipéridinyl-1)-2 quinazoline, composé décrit par la demanderesse dans sa demande de brevet n° 81/19767.

Les résultats du tableau II montrent clairement que les produits selon l'invention possèdent une activité antidépressive puissante. Cette activité est dans la plupart des cas plus puissante que celle de l'imipramine et s'accompagne d'une toxicité et d'effets secondaires de type cholinergique nettement inférieurs à ceux de l'imipramine.

Par ailleurs, on peut noter que le produit CM 40 331 étudié en comparaison est pratiquement inactif sur les tests pratiqués.

Par suite, les produits selon l'invention peuvent être utilisés en thérapeutique humaine pour le traitement de troubles neuropsychiques tels que les états dépressifs endogènes, réactionnels ou névrotiques ainsi que dans la dépression d'involution du vieillard.

Les produits pourront être présentés sous les formes galéniques correspondant à la voie orale (comprimés, gélules, etc.) et à la voie parentérale (ampoules injectables).

La posologie, variable suivant les affections à traiter et la voie d'administration, sera progressive et se situera entre 50 et 300 mg par jour chez l'adulte.

A titre d'exemple, on peut indiquer la préparation galénique suivante contenant un produit de l'invention :

| | |
|---|---|
| Gélule | |
| CM 40468 | 0,025 g |
| Amidon STA RX 1500 | 0,140 g |
| Aérosil 200 | 0,000 5 g |
| Stéarate de magnésium | 0,001 5 g |
| pour une gélule n° 3 | |

(Voir tableaux pages suivantes)

Tableau I

| Exemple n° | n° code du produit | $R_3$ | $R_4$ | $R_5$ | Base ou sel Point de fusion (solvant) |
|---|---|---|---|---|---|
| 2 | 40460 | Cl | $CH_3$ | Cl | Chlorhydrate<br>F > 260°C (méthanol) |
| 3 | 40468 | Cl | $CH_3$ | F | Base<br>F : 146-148°C(méthanol) |
| 4 | 40508 | $NO_2$ | $CH_3$ | F | Base<br>F : 220-222°C(éthanol) |
| 5 | 41125 | Cl | H | F | Chlorhydrate<br>F > 260°C(isopropanol) |
| 6 | 41128 | Cl | $-CH_2CH_2CH_3$ | F | Base<br>130-132°C(éthanol) |

Tableau II

| Produits n° | Toxicité | Test de Porsolt (dose en mg/kg) | Antagonisme de la ptôse a la réserpine DE 50 (mg/kg) | Antagonisme de l'hypothermie à la réserpine (à 10mg/kg) | Potentialisation de la yohimbine DE 50 (mg/kg) | Antagonisme de l'oxotrémorine DE 50 (mg/kg) |
|---|---|---|---|---|---|---|
| 40 498 | Atoxique à 1g/kg | - 31%** (10) | 2,8 | + 68%** | 7,8 | 43,5 |
| 40 460 | DL 80 = 1g/kg | - 11%* (10) | 2,1 | + 71%** (à 20mg/kg) | 5,4 | 142 |
| 40 468 | Atoxique à 1g/kg | - 61%** (10) | 0,98 | + 73%** | 1,65 | 50 |
| 41 125 | DL 40 = 1g/kg | - 46** (5) | 0,48 | + 81%** | 0,74 | 9,5 |
| 41 128 | Atoxique à 1g/kg | - 31** (5) | 6,2 | + 62%** | 1,7 | - |
| Imipramine | DL 50 = 453mg/kg | - 30%** (10) | 2,4 | + 59%** | 9,1 | 12 |
| 40 331 | Atoxique à 500mg/kg | - | 0% à 100mg/kg | - 20% à 100mg/kg | 10% à 100mg/kg | - |

* $p < 0{,}05$ ** $p < 0{,}01$

**Revendications** (pour les Etats contractants : BE, CH, DE, FR, GB, IT, LI, LU, NL, SE)

1. Dérivés de la pipérazinyl-2 phényl-4 quinazoline, caractérisés en ce qu'ils répondent à la formule générale :

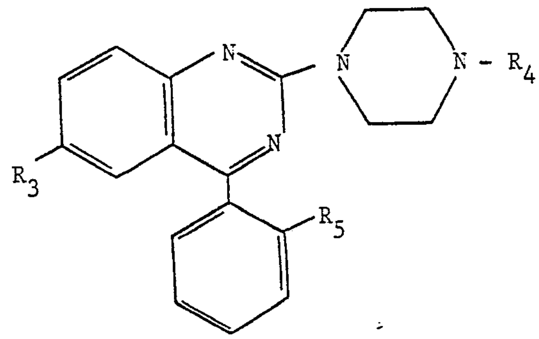

(II)

dans laquelle :

$R_3$ désigne un halogène, de préférence le chlore ou un groupe nitro,

$R_4$ représente H ou un groupe alkyle ayant de 1 à 4 atomes de carbone,

$R_5$ représente un halogène, de préférence le chlore et le fluor, et les sels desdits dérivés avec des acides.

2. Dérivés selon la revendication 1, caractérisés en ce que $R_3 = Cl$, $R_4 = CH_3$ et $R_5$ est Cl ou F.

3. Dérivés selon la revendication 1, caractérisés en ce que $R_3 = NO_2$, $R_4 = CH_3$ et $R_5$ est Cl ou F.

4. Dérivés selon la revendication 1, caractérisés en ce que $R_3 = Cl$, $R_5 = F$ et $R_4$ est l'hydrogène ou le groupe $-CH_2-CH_2-CH_3$.

5. Procédé de préparation des produits selon la revendication 1, caractérisé en ce que l'on utilise comme produit de départ une quinazolone de formule :

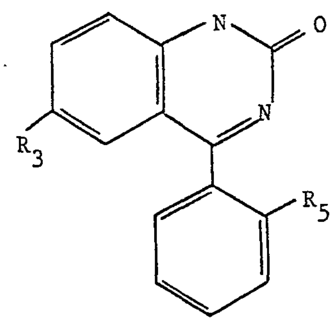

dans laquelle $R_3$ et $R_5$ ont la signification ci-dessus, que l'on fait réagir sur cette quinazolone un dérivé chloré du phosphore, de préférence l'oxychlorure de phosphore et que l'on fait réagir, au sein d'un solvant inerte, sur le dérivé chloré ainsi obtenu, une amine de formule :

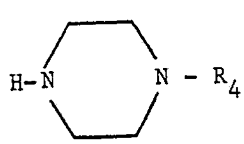

dans laquelle $R_4$ a la signification ci-dessus, ladite amine étant utilisée en excès.

6. Médicaments, caractérisés en ce qu'ils contiennent au moins un produit selon l'une quelconque des revendications 1 à 4.

7. Médicaments selon la revendication 6, caractérisés en ce qu'ils sont conditionnés en vue d'une administration par voie orale ou par voie parentérale.

**Revendications** (pour l'Etat contractant AT)

1. Procédé pour l'obtention de dérivés de la pipérazinyl-2 phényl-4 quinazoline répondant à la formule générale :

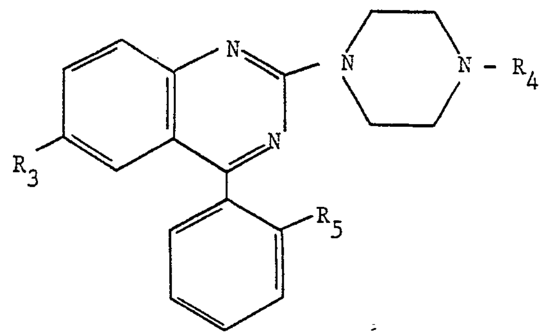

(II)

dans laquelle :

$R_3$ désigne un halogène, de préférence le chlore ou un groupe nitro,

$R_4$ représente H ou un groupe alkyle ayant de 1 à 4 atomes de carbone,

$R_5$ représente un halogène, de préférence le chlore et le fluor, et les sels desdits dérivés avec des acides, caractérisé en ce qu'on utilise comme produit de départ une quinazolone de formule :

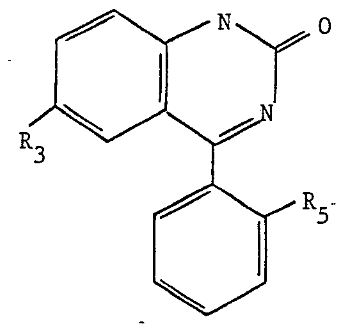

(1)

dans laquelle $R_3$ et $R_5$ ont la signification ci-dessus, que l'on fait réagir sur cette quinazolone un dérivé chloré du phosphore, de préférence l'oxychlorure de phosphore et que l'on fait réagir, au sein d'un solvant inerte, sur le dérivé chloré ainsi obtenu, une amine de formule : .

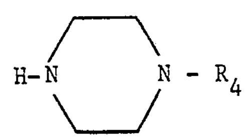

(3)

dans laquelle $R_4$ a la signification ci-dessus, ladite amine étant utilisée en excès.

2. Procédé selon la revendication 1, caractérisé en ce que l'on utilise comme produit de départ une quinazolone de formule (1) dans laquelle $R_3$ = Cl et $R_5$ est Cl ou F et une amine de formule (3) dans laquelle $R_4$ est le groupe $CH_3$.

3. Procédé selon la revendication 1, caractérisé en ce que l'on utilise comme produit de départ une quinazolone de formule (1) dans laquelle $R_3 = NO_2$, $R_5$ est Cl ou F et une amine de formule (3) dans laquelle $R_4$ est le groupe $CH_3$.

4. Procédé selon la revendication 1, caractérisé en ce que l'on utilise comme produit de départ une quinazolone de formule (1) dans laquelle $R_3$ = Cl et $R_5$ = F et une amine de formule (3) dans laquelle $R_4$ est l'hydrogène ou le groupe $-CH_2-CH_2-CH_3$.

**Claims** (for the contracting states : BE, CH, DE, FR, GB, IT, LI, LU, NL, SE)

1. Derivatives of 2-piperazinyl 4-phenyl quinazoline, characterized in that they respond to the general formula :

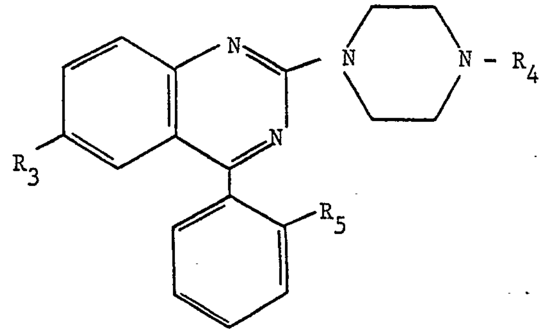

(II)

in which :

$R_3$ designates a halogen, preferably chlorine or a nitro group,

$R_4$ represents H or an alkyl group of 1 to 4 carbon atoms,

$R_5$ represents a halogen, preferably chlorine and fluorine, and salts of said derivatives with acids.

2. Derivatives according to claim 1, characterized in that $R_3 = Cl$, $R_4 = CH_3$ and $R_5$ is Cl or F.

3. Derivatives according to claim 1, characterized in that $R_3 = NO_2$, $R_4 = CH_3$ and $R_5$ is Cl or F.

4. Derivatives according to claim 1, characterized in that $R_3 = Cl$, $R_5 = F$ and $R_4$ is hydrogen or the $-CH_2-CH_2-CH_3$ group.

5. Process for preparing the products according to claim 1, characterized in that a quinazolone of the following formula is used as starting product :

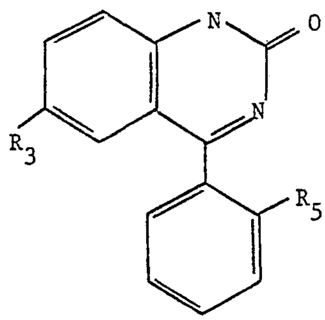

in which $R_3$ and $R_5$ have the above meaning, in that a chlorinated derivative of phosphorus, preferably phosphorus oxychloride, is caused to react on this quinazolone, and in that an amine of the following formula is caused to react in an inert solvent on the chlorinated derivative thus obtained :

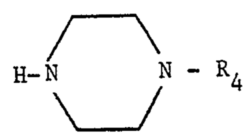

in which $R_4$ has the above meaning, said amine being used in excess.

6. Drugs, characterized in that they contain at least a product according to any one of claims 1 to 4.

7. Drugs according to claim 6, characterized in that they are packed for administration by the oral route of the parenteral route.

**Claims** (for the contracting state AT)

1. Process for obtaining derivatives of 2-piperazinyl 4-phenyl quinazoline responding to the general formula :

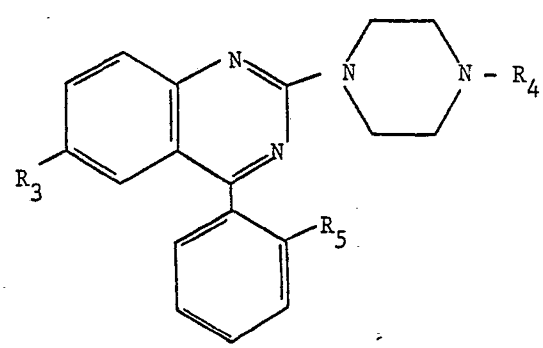

(II)

in which

$R_3$ designates a halogen, preferably chlorine or a nitro group,

$R_4$ is H or an alkyl group of 1 to 4 carbon atoms,

$R_5$ is a halogen, preferably chlorine and fluorine, and the salts of said derivatives with acids, characterized in that a quinazolone of the following formula is used as starting product :

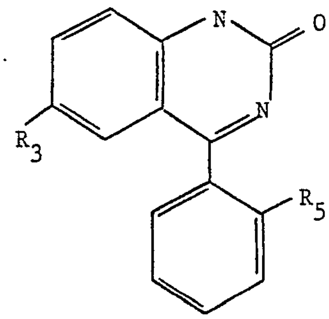

(1)

in which $R_3$ and $R_5$ have the above meaning, in that a chlorinated derivate of phosphorus, preferably phosphorus oxychloride, is caused to react on this quinazolone, and in that an amine of the following formula is caused to react in an inert solvent on the chlorinated derivative thus obtained :

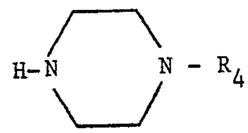

(3)

in which $R_4$ has the above meaning, said amine being used in excess.

2. Process according to claim 1, characterized in that a quinazolone of formula (1) is used as starting product, in which $R_3$ = Cl and $R_5$ is Cl or F and an amine of formula (3) in which $R_4$ is the $CH_3$ group.

3. Process according to claim 1, characterized in that a quinazolone of formula (1) is used as starting product in which $R_3$ = $NO_2$, $R_5$ is Cl or F, and an amine of formule (3) in which $R_4$ is the $CH_3$ group.

4. Process according to claim 1, characterized in that a quinazolone of formula (1) is used as starting product in which $R_3$ = Cl and $R_5$ = F and an amine of formula (3) in which $R_4$ is hydrogen or the $-CH_2-CH_2-CH_3$ group.

**Patentansprüche** (für die Vertragsstaaten BE, CH, DE, FR, GB, IT, LI, LU, NL, SE)

1. 2-Piperazinyl-4-phenyl-chinazolin-Derivate, dadurch gekennzeichnet, daß sie der allgemeinen Formel

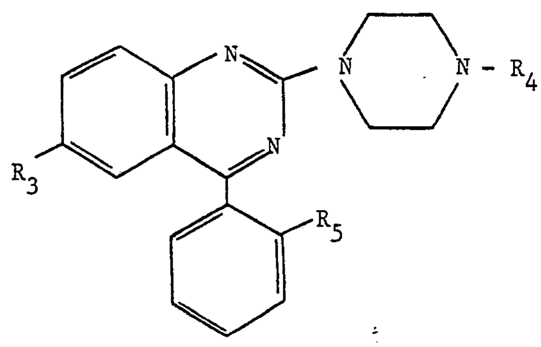

(II)

entsprechen, worin :

$R_3$ ein Halogen, vorzugsweise Chlor oder eine Nitrogruppe bezeichnet,

$R_4$ H oder eine Alkylgruppe mit 1 bis 4 Kohlenstoffatomen darstellt,

$R_5$ für ein Halogen, vorzugsweise Chlor und Fluor, steht, und die Salze dieser Derivate mit Säuren.

2. Derivate nach Anspruch 1, dadurch gekennzeichnet, daß $R_3$ = Cl, $R_4$ = $CH_3$ und $R_5$ = Cl oder F.

3. Derivate nach Anspruch 1, dadurch gekennzeichnet, daß $R_3$ = $NO_2$, $R_4$ = $CH_3$ und $R_5$ = Cl oder F.

4. Derivate nach Anspruch 1, dadurch gekennzeichnet, daß $R_3$ = Cl, $R_5$ = F und $R_4$ = Wasserstoff oder die Gruppe $-CH_2-CH_2-CH_3$.

5. Verfahren zur Herstellung der Produkte nach Anspruch 1, dadurch gekennzeichnet, daß man als Ausgangsprodukt ein Chinazolon der Formel

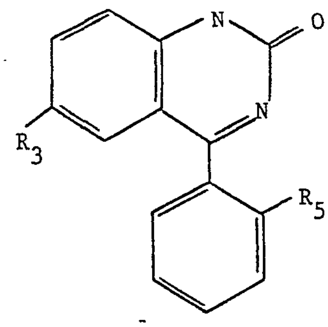

worin $R_3$ und $R_5$ obige Bedeutung haben, verwendet, daß man dieses Chinazolon mit einem chlorierten Phosphorderivat, vorzugsweise Phosphoroxychlorid, zur Umsetzung bringt und daß man das so erhaltene chlorierte Derivat in einem inerten Lösungsmittel mit einem Amin der Formel

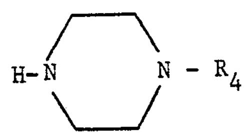

worin $R_4$ obige Bedeutung hat, zur Umsetzung bringt, wobei das Amin im Überschuß eingesetzt wird.

6. Medikamente, dadurch gekennzeichnet, daß sie mindestens ein Produkt nach einem der Ansprüche 1 bis 4 enthalten.

7. Medikamente nach Anspruch 6, dadurch gekennzeichnet, daß sie zur oralen oder zur parenteralen Verabreichung konditioniert sind.

**Patentansprüche** (für den Vertragsstaat AT)

1. Verfahren zur Herstellung von 2-Piperazinyl-4-phenylchinazolin-Derivaten der allgemeinen Formel

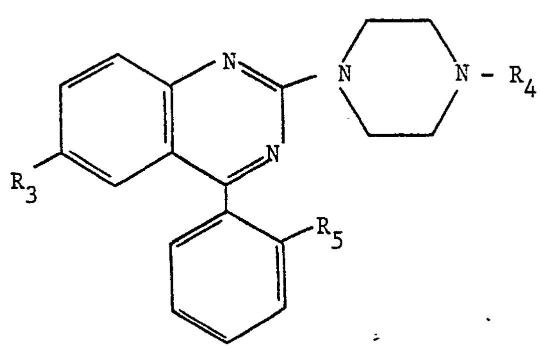

(II)

worin :

$R_3$ ein Halogen, vorzugsweise Chlor oder eine Nitrogruppe bezeichnet,

$R_4$ H oder eine Alkylgruppe mit 1 bis 4 Kohlenstoffatomen darstellt,

$R_5$ für ein Halogen, vorzugsweise Chlor und Fluor, steht, und die Salze dieser Derivate mit Säuren, dadurch gekennzeichnet, daß man als Ausgangsprodukt ein Chinazolon der Formel

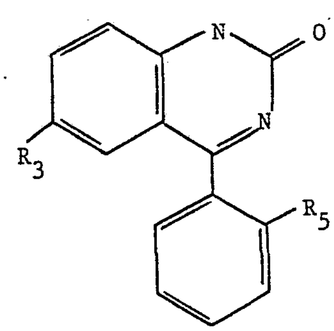

worin $R_3$ und $R_5$ obige Bedeutung haben, verwendet, daß man dieses Chinazolon mit einem chlorierten Phosphorderivat, vorzugsweise Phosphoroxychlorid, zur Umsetzung bringt und daß man das so erhaltene chlorierte Derivat in einem inerten Lösungsmittel mit einem Amin der Formel

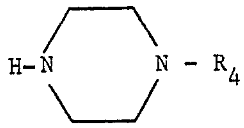

(3)

worin $R_4$ obige Bedeutung hat, zur Umsetzung bringt, wobei das Amin in Überschuß eingesetzt wird.

2. Verfahren nach Anspruch 1, dadurch gekennzeichnet, daß man als Ausgangsprodukt ein Chinazolon der Formel (1), worin $R_3$ = Cl und $R_5$ = Cl oder F, und ein Amin der Formel (3), worin $R_4$ die Gruppe $CH_3$ ist, verwendet.

3. Verfahren nach Anspruch 1, dadurch gekennzeichnet, daß man als Ausgangsprodukt ein Chinazolon der Formel (1), worin $R_3$ = $NO_2$, $R_5$ = Cl oder F, und ein Amin der Formel (3), worin $R_4$ die Gruppe $CH_3$ ist, verwendet.

4. Verfahren nach Anspruch 1, dadurch gekennzeichnet, daß man als Ausgangsprodukt ein Chinazolon der Formel (1), worin $R_3$ = Cl und $R_5$ = F, und ein Amin der Formel (3), worin $R_4$ Wasserstoff oder die Gruppe $—CH_2—CH_2—CH_3$ ist, verwendet.